(19) Europäisches Patentamt
European Patent Office
Office européen des brevets

(11) EP 1 179 524 A1

# (12) EUROPEAN PATENT APPLICATION

(43) Date of publication:
**13.02.2002 Bulletin 2002/07**

(51) Int Cl.[7]: **C07C 67/37**, C07C 69/675, C07D 315/00

(21) Application number: **00306838.4**

(22) Date of filing: **10.08.2000**

(84) Designated Contracting States:
**AT BE CH CY DE DK ES FI FR GB GR IE IT LI LU MC NL PT SE**
Designated Extension States:
**AL LT LV MK RO SI**

(71) Applicant: **Kvaerner Process Technology Limited London W2 6LE (GB)**

(72) Inventors:
- **Crabtree, Simon Peter**
  **Newton Hall, Durham DH1 5NA (GB)**
- **Henderson, Richard Kevin**
  **Netherby Rise, Darlington DL3 8Se (GB)**
- **Walker, Andrew James**
  **Oxbridge, Stockton-on-Tees TS18 4DL (GB)**
- **Willett, Paul**
  **Witton Le Wear, County Durham DL14 0AH (GB)**

(74) Representative: **Eyles, Christopher Thomas**
**W.P. THOMPSON & CO. Celcon House 289-293 High Holborn**
**London WC1V 7HU (GB)**

## (54) Process for the carbonylation of oxiranes

(57) A process is described for the carbonylation of an oxirane, such as ethylene oxide, which comprises reacting the oxirane under carbonylation conditions with carbon monoxide in a solvent, such as alkanol, for example methanol, in the presence of a cobalt catalyst and of an N-alkylated azole promoter, such as 1-methylpyrazole, and recovering the resulting carbonylation product, such as an alkyl ester of 3-hydroxypropionic acid, for example methyl 3-hydroxypropionate.

EP 1 179 524 A1

## Description

This invention relates to a process for the carbonylation of oxiranes.

Propane-1,3-diol is used as an intermediate in the production of polyesters for production of fibres or films. It can be produced from glycerol using recombinant bacteria expressing recombinant diol dehydratase. Such a process is taught in United States Patent Specification No. 5,821,092.

It has also been proposed to subject acrolein to hydration so as to form 3-hydroxypropanal which is then hydrogenated to produce propane-1,3-diol. In this connection reference may be made to United States Patent Specification No. 5,364,987.

Both glycerol and acrolein are, however, generally relatively expensive. Accordingly methods have been developed for production of propane-1,3-diol using the more readily available and cheaper starting material, ethylene oxide.

Thus it is known to effect carbonylation of an oxirane, such as ethylene oxide, to produce a precursor suitable for conversion to a diol, such as propane-1,3-diol. For example, a two-step process has been proposed for the production of propane-1,3-diol, in which ethylene oxide is subjected to an oxonation reaction followed by hydrogenation:

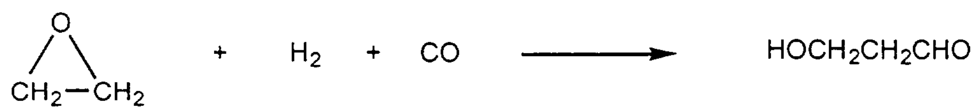

$$HOCH_2CH_2CHO + H_2 \rightarrow HOCH_2CH_2CH_2OH$$

United States Patent Specification No. 5,981,808 describes the use of a non-phosphine-ligated cobalt compound as oxonation catalyst in an essentially non-water-miscible solvent followed by water extraction to separate the catalyst from the 3-hydroxypropanal produced as oxonation product. The aqueous mixture containing the 3-hydroxypropanal is then subjected to hydrogenation.

United States Patent Specification No. 5,585,528 proposes addition of a lipophilic tertiary amine as a promoter in such a process.

Use of methyl t-butyl ether for extraction of the aqueous mixture to recover cobalt catalyst for re-use is described in United States Patent Specification No. 5,770,776.

United States Patent Specification No. 5,786,524 teaches a similar process and proposes the use of a rhodium catalyst as an alternative catalyst in the oxonation step.

It has also been proposed to combine the oxonation and hydrogenation steps into a one-step process with, it is claimed, minimal production of 3-hydroxypropanal as byproduct. Such a one-step process can be effected using a phosphine complex of cobalt carbonyl as the major catalyst ingredient. However, the use of a ruthenium compound as catalyst has also been proposed. An organic solvent is used in the reaction enabling a water extraction to be used in order to separate propane-1,3-diol from the oxonation mixture. Ethylene oxide conversions of 55% with a selectivity towards propane-1,3-diol of 87% are reported.

United States Patents Nos. 5,310,948 and 5,359,081 teach formation of β-propiolactone or polymers thereof by reaction of carbon monoxide and ethylene oxide in the presence of a cobalt-containing catalyst system comprising a source of cobalt and a hydroxyl-substituted pyridine. The reaction is:

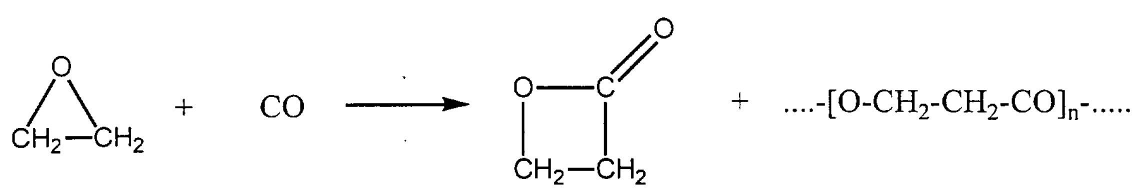

United States Patent Specification No. 3,260,738 proposes a process for the production of hydracrylate esters, such as methyl 3-hydroxypropionate, by carbonylation of ethylene oxide using a cobalt carbonyl catalyst. A primary monohydric alcohol is used as solvent and reactant with or without the presence of a hydrocarbon co-solvent. A ligand promoter may be used to stabilise the metal of the catalyst. A co-catalyst may be used, preferred co-catalysts being tertiary amines, such as pyridine, N,N-benzyldimethylamine, and N-methylpyrrolidine.

United States Patent Specification No. 5,731,255 discloses a catalytic system for carbonylation of an olefinic or acetylenic compound, such as propyne, which comprises a Group VIII metal source supported on a carrier, a ligand, and an acid. Cobalt is mentioned as a suitable Group VIII metal, while the ligand can be a phosphine, such as triphenylphosphine. It is suggested that an electron donative compound can optionally be added. Amongst a long list of possible electron donative compounds there are mentioned imidazole and 1-methylimidazole, although no experimental results using these compounds are given. This document also discloses an alternative form of catalyst which comprises a Group VIII metal (except palladium), a ligand, such as a phosphine, an electron donative compound, and optionally an acid.

United States Patent Specification No. 4,407,726 proposes preparation of a carboxylic acid, such as propionic acid, by carbonylation of an olefin, such as ethylene, in the presence of water by the use of a molybdenum-nickel or tungsten-nickel co-catalyst in the presence of a promoter comprising an organo-phosphorus compound or an organo-nitrogen compound wherein the phosphorus and nitrogen are trivalent and in the presence of a halide. Imidazole is included in a list of organo-nitrogen compounds but no experimental results are given using imidazole as promoter.

In United States Patent Specification No. 5,442,107 there is described a process for preparing a carboxylic acid having (n + 1) carbon atoms (for example, acetic acid) from an alkanol having n carbon atoms (for example, methanol) by liquid phase, rhodium catalysed carbonylation. The process is stabilised by using a catalyst stabiliser such as the quaternised forms of 4-methylimidazole and 2-ethyl-4-methylimidazole. Evidence is also provided that too much precipitation of rhodium occurs when the quaternised forms of N-methylimidazole, imidazole, 2-ethylimidazole, benzimidazole, or 1,2-dimethylimidazole are used.

Japanese Published Patent Specification No. 63170338 discloses a method of producing a malonic ester by the cobalt catalysed carbonylation of dichloromethane by reaction with carbon monoxide and an alcohol in the presence of imidazole as a promoter. Under the reaction conditions used the promoter is converted to the corresponding quaternary ammonium iodide.

It is an objective of the present invention to provide an improved process for the production of precursors suitable for subsequent conversion to propane-1,3-diol. In addition the present invention seeks to provide an improved process for effecting carbonylation of an oxirane in the presence of a solvent, such as an alkanol.

According to the present invention there is provided a process for the carbonylation of an oxirane which comprises reacting the oxirane under carbonylation conditions with carbon monoxide in a solvent in the presence of a cobalt catalyst and of an N-alkylated azole promoter, and recovering the resulting carbonylation product.

The solvent is non-aqueous and may comprise an alkanol, preferably a substantially anhydrous alkanol, in which case the carbonylation product comprises an alkyl ester of an optionally substituted 3-hydroxypropionic acid.

Alternatively the solvent can be an inert aprotic solvent, such as a hydrocarbon or an ether, for example, 1,2-dimethoxyethane, in which case the product is a β-lactone.

The carbonylation product thus comprises a cyclic or linear ester of an optionally substituted 3-hydroxypropionic acid.

As examples of N-alkylated azoles which can be used in the process of the invention there can be mentioned pyrazoles of the formula:

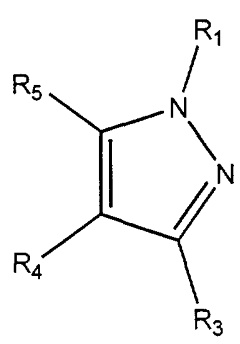

(I)

and imidazoles of the formula:

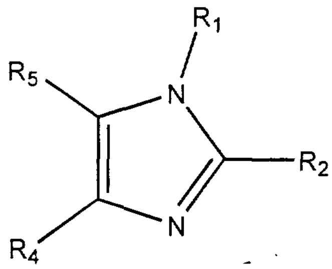

(II)

In the above formulae (I) and (II)

$R_1$ is an alkyl group;
$R_2$ is a hydrogen atom, a hydroxy group, an alkyl group, a hydroxyalkyl group, an alkoxy group, an alkoxycarbonyl group, an aryl group, or an optionally substituted amide group;
$R_3$ is a hydrogen atom, a hydroxy group, an alkyl group, a hydroxyalkyl group, an alkoxy group, an alkoxycarbonyl group, an aryl group, or an optionally substituted amide group;
$R_4$ is a hydrogen atom, a hydroxy group, an alkyl group, a hydroxyalkyl group, an alkoxy group, an alkoxycarbonyl group, an aryl group, or an optionally substituted amide group; and
$R_5$ is a hydrogen atom, a hydroxy group, an alkyl group, a hydroxyalkyl group, an alkoxy group, an alkoxycarbonyl group, an aryl group, or an optionally substituted amide group; or
$R_4$ and $R_5$, together with the atoms to which they are attached, form an optionally substituted carbocyclic or heterocyclic ring system.

In formulae (I) and (II) each alkyl or alkoxy group preferably contains from 1 to about 6 carbon atoms, more preferably 1 or 2 carbon atoms, and may be a straight chain group or a branched chain group. Suitable alkyl groups include methyl, ethyl, n-propyl, iso-propyl, n-butyl, isobutyl, t-butyl, and pentyl, and the like, while suitable alkoxy groups include methoxy, ethoxy, n-propoxy, iso-propoxy, n-butoxy, iso-butoxy, t-butoxy, and pentoxy, and the like.
Examples of suitable aryl groups include phenyl, o-tolyl, m-tolyl, p-tolyl, o-methoxyphenyl, m-methoxyphenyl, p-methoxyphenyl, o-ethoxyphenyl, m-ethoxyphenyl, p ethoxyphenyl, naphthyl-1, and naphthyl-2, and the like.
Suitable optionally substituted amide groups include $-CONH_2$, $-CONH(CH_3)_)$, $-CON(CH_3)_2$, $-CONHCH_2CH_3$, $-CON(CH_2CH_3)_2$, and $-CONHC_6H_5$, and the like.
Preferred N-substituted azoles include N-alkylated pyrazoles, N-alkylated imidazoles, N-alkylated benzimidazoles, and N-alkylated benzopyrazoles, and the like.
Specific N-alkylated azoles which can be used include 1-methylpyrazole, 1-ethylpyrazole, 1-iso-propylpyrazole, 1n-butylpyrazole, t-butylpyrazole, 1-pentylpyrazole, 1,3,5-trimethylpyrazole, 1-methylimidazole, 1-ethylimidazole, 1-iso-propylimidazole, 1-n-butylimidazole, 1-t-butylimidazole, 1-pentylimidazole, and the like.
The oxirane is preferably an optionally substituted oxirane of the formula:

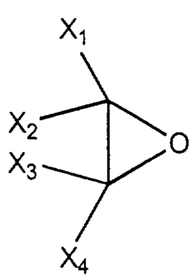

(III)

wherein

$X_1$ is a hydrogen atom, an alkyl group, or a phenyl group;
$X_2$ is a hydrogen atom, an alkyl group, or a phenyl group;
$X_3$ is a hydrogen atom, an alkyl group, or a phenyl group; and
$X_4$ is a hydrogen atom, an alkyl group, or a phenyl group; or

$X_1$ and $X_3$, or $X_2$ and $X_4$, together form a five membered or six membered carbocyclic or heterocyclic ring.

Particularly preferred oxiranes are ethylene oxide, propylene oxide, styrene oxide, 1,2-epoxyhexane, 1,2-epoxyoctane, and cyclopentene oxide, and the like.

As alkanol solvent there is preferably used an alkanol which contains from 1 to about 6 carbon atoms. Preferred. alkanols include methanol, ethanol, n-propanol, iso-propanol, and n-butanol, and the like.

The carbonylation conditions preferably include use of a pressure in the range of from about 200 psig (about 1.38 MPa gauge) to about 3000 psig (about 20.68 MPa gauge), for example from about 800 psig (about 5.52 MPa gauge) to about 1200 psig (about 8.27 MPa gauge). Preferably also the carbonylation conditions include use of a temperature in the range of from about 50°C to about 150°C, for example from about 60°C to about 100°C. More preferably the pressure is in the range of from about 200 psig (about 1.38 MPa gauge) to about 900 psig (about 6.21 MPa gauge). More preferably also the temperature is in the range of from about 85°C to about 100°C.

The ratio of the N-alkylated azole to cobalt preferably is within the range of from about 0.1 moles to about 200 moles, more preferably from about 1 moles to about 5 moles, of N-alkylated azole promoter per mole of cobalt.

The cobalt can be introduced into the reaction in the form of a source of cobalt, such as cobalt (II) acetate, cobalt carbonyl, cobalt hydroxide, cobalt acetyl acetonate, cobalt nitrate, and the like, which can be converted into a catalytic species effective for the desired carbonylation reaction. This catalytically active species is believed to be derived from dicobalt octacarbonyl, $Co_2(CO)_8$, under moderate carbon monoxide pressure.

In the process of the invention it is possible to use dicobalt octacarbonyl or another cobalt carbonyl but it is well known that the use of cobalt carbonyls requires special safety and handling measures, particularly in commercial units. However, other cobalt salts, such as cobalt acetate, cobalt hydroxide, cobalt acetyl acetonate, cobalt nitrate, and the like, can alternatively be used as a catalyst precursor because they can be readily carbonylated in the presence of carbon monoxide under elevated pressure. It is preferred to effect the catalyst preparation step in the absence of the oxirane because it then becomes possible during conversion of a cobalt salt to an active cobalt carbonyl catalytic species to use a higher temperature than is optimal for carbonylation of the oxirane.

The active catalytic species is normally soluble in the reaction medium so that it can be used in a homogeneous reaction. However, it can alternatively be absorbed or adsorbed on a solid carrier, thereby making a gaseous reaction possible.

Apart from the cobalt carbonyl catalytic species and the N-alkylated azole, no other catalyst components are required. In particular, no triphenylphosphine or other ligand needs to be utilised in the process of the invention.

In the catalyst preparation step the source of cobalt is preferably added in the form of a pre-formed complex with the N-alkylated azole.

In a particularly preferred process the oxirane is ethylene oxide, the solvent is methanol, and the resulting carbonylation product comprises methyl 3-hydroxypropionate. If methanol is replaced by another alkanol, for example n-propanol, then the corresponding ester, for example n-propyl 3-hydroxypropionate, is produced. If the oxirane is propylene oxide and the alkanol is methanol, then the product comprises a mixture of methyl 4-hydroxybutyrate and methyl 3-hydroxy-2-methylpropionate.

The products of the process may be recovered from the reaction product mixture by conventional methods, such as fractional distillation or extractive distillation. Addition of a solvent, such as di-n-butyl phthalate, may aid in the product recovery step or steps by providing a medium for retention of the catalyst once the product or products and any remaining reactants have been removed.

The invention is further illustrated in the following Examples.

Example 1

A 1 litre autoclave was purged with about 50 litres (measured at 0°C and 100 kPa) of carbon monoxide. In a separate container 3.3 g of cobalt acetate tetrahydrate (0.0132 moles) was dissolved in 500 ml of methanol and then 4.4 g of 1-methylpyrazole (0.053 moles) was added to the resultant solution with stirring. This methanolic solution was then loaded into the autoclave, stirred at 600 to 800 rpm, pressurised to 500 psig (3.45 MPa gauge) with carbon monoxide and heated to 185°C whereupon a pressure of about 1000 psig (about 6.89 MPa gauge) developed. After two hours the autoclave was cooled to 85°C and the autoclave pressure was set to 900 psig (6.21 MPa gauge) with carbon monoxide. 100 g of ethylene oxide was then pumped into the autoclave. During the course of the reaction the pressure was maintained at 900 psig (6.21 MPa gauge) by the addition of carbon monoxide. After 12 hours the autoclave was cooled and the gases were gently vented. The product and catalyst residue were recovered as a deep red liquid. This liquid was analysed by gas chromatography using a Perkin-Elmer gas chromatograph having a split injector and a flame ionisation detector with helium as carrier gas at a pressure of 12 psig (82.74 kPa gauge) and with a CP Sil 8CB column 50 m long with a diameter of 0.32 mm and with a phase thickness of 1.2 μm. A temperature programme was used which consisted of the following steps:

1. hold at 40°C for 15 minutes;
2. heat at 8°C per minute to 160°C;
3. hold at 160°C for 5 minutes;
4. heat at 30°C per minute to 280°C; and
5. hold at 280°C for 20 minutes.

The results set out in the Table were obtained.

Example 2

The procedure of Example 1 was repeated using 0.05 moles of 1-methylimidazole in place of the 0.053 moles of 1-methylpyrazole. The results obtained are set out in the Table.

Example 3

Instead of using 1-methylpyrazole in the procedure of Example 1, an equivalent amount of 1,3,5-trimethylpyrazole was used with the results listed in the Table.

Example 4 (Comparative)

The procedure of Example 1 was repeated but no promoter, such as 1-methylpyrazole, was added. Again, the results are given in the Table. The selectivity to methyl 3-hydroxypropionate dropped significantly.

Example 5 (Comparative)

Following the same procedure used in Example 1, but using pyrazole in place of 1-methylpyrazole, little or no methyl 3-hydroxypropionate was observed in the product. Instead the major products were methyl formate and 2-methoxyethanol.

Example 6 (Comparative)

When imidazole was used in place of 1-methylpyrazole in the procedure of Example 1, there was a significant drop in the selectivity of the reaction to the desired carbonylation product, methyl 3-hydroxypropionate. As in Example 5, the major products were methyl formate and 2-methoxyethanol.

Example 7 (Comparative)

Upon replacing 1-methylpyrazole in the procedure of Example 1 by 2-methylimidazole it was again observed that little or no methyl 3-hydroxypropionate was formed, the major products being methyl formate and 2-methoxyethanol.

TABLE

| Example | Promoter | Wt % Selectivity to HMP | Wt % of HCOOMe |
|---|---|---|---|
| 1 | 1-methylpyrazole | 71 | <1 |
| 2 | 1-methylimidazole | 58 | <1 |
| 3 | 1,3,5-trimethylpyrazole | 69 | <1 |
| 4 | No promoter | 45 | <1 |
| 5 | pyrazole | <2 | 41 |
| 6 | imidazole | <2 | 49 |
| 7 | 2-methylimidazole | <2 | 30 |
| Notes: 1. HMP means methyl 3-hydroxypropionate<br>2. HCOOMe means methyl formate | | | |

It will be observed from these results that it is important that the azole promoter used bears an N-alkyl substituent. In Comparative Examples 5 to 7, in which no N-alkyl substituent is present in the azole promoter, the selectivity to the desired product, methyl 3-hydroxypropionate, was less than 2 %. On the other hand significantly higher selectivities to methyl 3-hydroxypropionate are observed when an N-alkylated azole is used, as is demonstrated by Examples 1 to 3.

## Claims

1. A process for the carbonylation of an oxirane which comprises reacting the oxirane under carbonylation conditions with carbon monoxide in a solvent in the presence of a cobalt catalyst and of an N-alkylated azole promoter, and recovering the resulting carbonylation product.

2. A process according to claim 1, wherein the oxirane is an optionally substituted oxirane of the formula:

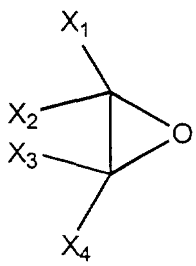

(III)

wherein

$X_1$ is a hydrogen atom, an alkyl group, or a phenyl group;
$X_2$ is a hydrogen atom, an alkyl group, or a phenyl group;
$X_3$ is a hydrogen atom, an alkyl group, or a phenyl group; and
$X_4$ is a hydrogen atom, an alkyl group, or a phenyl group; or
$X_1$ and $X_3$, or $X_2$ and $X_4$, together form a five membered or six membered carbocyclic or heterocyclic ring.

3. A process according to claim 2, wherein the oxirane is selected from ethylene oxide, propylene oxide, styrene oxide, 1,2-epoxyhexane, 1,2-epoxyoctane, and cyclopentene oxide.

4. A process according to any one of claims 1 to 3, wherein the carbonylation conditions include use of a pressure in the range of from about 200 psig (about 1.38 MPa gauge) to about 3000 psig (about 20.68 MPa gauge) and of a temperature in the range of from about 50°C to about 150°C.

5. A process according to claim 4, wherein the pressure is in the range of from about 200 psig (about 1.38 MPa gauge) to about 900 psig (about 6.21 MPa gauge) and the temperature is in the range of from about 60°C to about 100°C.

6. A process according to any one of claims 1 to 5, wherein the promoter is selected from pyrazoles of the formula:

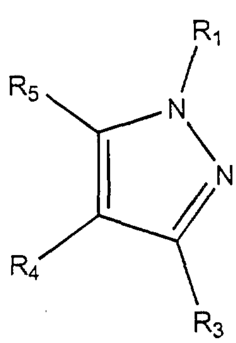

(I)

and imidazoles of the formula:

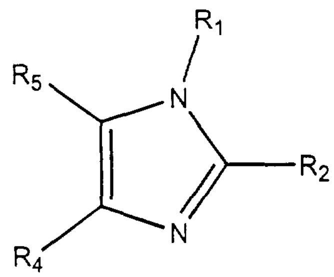

(II)

wherein

$R_1$ is an alkyl group;
$R_2$ is a hydrogen atom, a hydroxy group, an alkyl group, a hydroxyalkyl group, an alkoxy group, an alkoxycarbonyl group, an aryl group, or an optionally substituted amide group;
$R_3$ is a hydrogen atom, a hydroxy group, an alkyl group, a hydroxyalkyl group, an alkoxy group, an alkoxycarbonyl group, an aryl group, or an optionally substituted amide group;
$R_4$ is a hydrogen atom, a hydroxy group, an alkyl group, a hydroxyalkyl group, an alkoxy group, an alkoxycarbonyl group, an aryl group, or an optionally substituted amide group; and
$R_5$ is a hydrogen atom, a hydroxy group, an alkyl group, a hydroxyalkyl group, an alkoxy group, an alkoxycarbonyl group, an aryl group, or an optionally substituted amide group; or
$R_4$ and $R_5$, together with the atoms to which they are attached, form an optionally substituted carbocyclic or heterocyclic ring system.

**7.** A process according to claim 6, wherein the promoter is selected from 1-methylpyrazole, 1-ethylpyrazole, 1-iso-propylpyrazole, 1-n-butylpyrazole, 1-t-butylpyrazole, 1-pentylpyrazole, 1,3,5-trimethylpyrazole, 1-methylimidazole, 1-ethylimidazole, 1-iso-propylimidazole, 1-n-butylimidazole, 1-t-butylimidazole, and 1-pentylimidazole.

**8.** A process according to any one of claims 1 to 7, wherein from about 0.1 moles to about 200 moles of N-alkylated azole promoter are present per mole of cobalt.

**9.** A process according to claim 8, wherein from about 1 moles to about 5 moles of N-alkylated azole promoter are present per mole of cobalt.

**10.** A process according to any one of claims 1 to 9, wherein the solvent comprises an alkanol and wherein the carbonylation product comprises an alkyl ester of an optionally substituted 3-hydroxy-propionic acid.

**11.** A process according to claim 10, wherein the alkanol is selected from methanol, ethanol, n-propanol, iso-propanol, and n-butanol.

**12.** A process according to claim 10 or claim 11, wherein the oxirane is ethylene oxide, the alkanol is methanol, and the resulting carbonylation product comprises methyl 3-hydroxypropionate.

**13.** A process according to any one of claims 1 to 9, wherein the solvent is an inert aprotic solvent and wherein the carbonylation product comprises a β-lactone.

**14.** A process according to claim 13, wherein the aprotic inert solvent is a hydrocarbon or an ether.

**15.** A process according to claim 13, wherein the aprotic inert solvent is 1,2-dimethoxyethane.

**16.** A process according to any one of claims 13 to 15, wherein the oxirane is ethylene oxide and wherein the carbonylation product comprises β-propiolactone.

European Patent Office

# EUROPEAN SEARCH REPORT

Application Number
EP 00 30 6838

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | EP 0 577 206 A (SHELL INTERNATIONALE RESEARCH MAATSCHAPPIJ B.V.) 5 January 1994 (1994-01-05) * page 2, line 27 - line 51 * * page 3 - page 4; examples * * page 5; claims * | 1 | C07C67/37 C07C69/675 C07D315/00 |

TECHNICAL FIELDS SEARCHED (Int.Cl.7)
C07C
C07D

The present search report has been drawn up for all claims

| Place of search | Date of completion of the search | Examiner |
|---|---|---|
| THE HAGUE | 3 January 2001 | Kinzinger, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 00 30 6838

This annex lists the patent family members relating to the patent documents cited in the above-mentioned European search report.
The members are as contained in the European Patent Office EDP file on
The European Patent Office is in no way liable for these particulars which are merely given for the purpose of information.

03-01-2001

| Patent document cited in search report | Publication date | Patent family member(s) | Publication date |
|---|---|---|---|
| EP 577206 A | 05-01-1994 | CA 2099200 A | 30-12-1993 |
| | | CN 1082540 A,B | 23-02-1994 |
| | | DE 69320556 D | 01-10-1998 |
| | | DE 69320556 T | 11-02-1999 |
| | | ES 2119856 T | 16-10-1998 |
| | | JP 6065223 A | 08-03-1994 |
| | | SG 49666 A | 15-06-1998 |
| | | US 5310948 A | 10-05-1994 |
| | | US 5359081 A | 25-10-1994 |

EPO FORM P0459

For more details about this annex : see Official Journal of the European Patent Office, No. 12/82